# EUROPEAN PATENT SPECIFICATION

(11) **EP 0 584 348 B1**
(45) Date of publication and mention of the grant of the patent: **18.05.2005**
(21) Application number: 93908340.8
(22) Date of filing: 10.03.1993
(51) Int. Cl.: C12N 15/86, C12N 15/87, A61K 39/12, C07H 15/12

(54) **GENETIC VACCINE FOR IMMUNODEFICIENCY VIRUSES**
GENETISCHER IMPFSTOFF GEGEN DEN IMMUNSCHWÄCHE VIRUS
VACCIN GENETIQUE POUR VIRUS D'IMMUNODEFIENCE

(30) Priority: 11.03.1992 US 850189
(43) Date of publication of application: 02.03.1994
(73) Proprietor: Powderject Vaccines, Inc., Madison, Wisconsin 53711 (US)
(72) Inventor: HAYNES, Joel, R., Middleton, WI 53562 (US)
(74) Representative: Woods, Geoffrey Corlett
(86) International application number: PCT/US1993/002338
(87) International publication number: WO 1993/017706

(56) References cited:
- EP-A- 0 213 894
- WO-A-89/05349
- WO-A-91/00359
- WO-A-91/07487
- US-A- 5 036 006
- MOL. IMMUNOL., vol. 28, no. 3, 1991 pages 231-234, HAYNES ET AL. 'Strategy for devrloping a genetically-engineered whole virus vaccine against HIV'
- J. VIROLOGY, vol. 65, no. 9, September 1991 pages 4555-4564, RHIM ET AL. 'Deletions in the tRNALys primer-binding site of Human Immunodeficiency Virus type 1 identify essential regions for reverse transcription'
- DATABASE WPI Week 9127 Derwent Publications Ltd., London, GB; AN 91-200880 & US-A-7 506 947 (NATL INSTITUTE OF HEALTH) , 28 May 1991
- J. VIROLOGY, vol. 65, no. 1, 1991 pages 532-536, POZNANSKY ET AL. 'Gene transfer into human lymphocytes by a defective human immunodoeficiency virus type 1 vector'
- Proceedings National Academy of Sciences, Volume 87, issued December 1990, YANG et al., "In vivo and In vitro Gene Transfer to Mammalian Somatic Cells by Particle Bombardment", pages 9568-9572, especially 9568-9569.
- Biotechnology News, Volume 11, No. 28, issued 21 November 1991, "Vical Gene Delivery Technique May Improve On HIV and Other Vaccines", see page 5.

## Description

### Field of the Invention

The present invention relates to the general field of genetic vaccines and relates, in particular, to genetic vaccines for immunodeficiency viruses.

### Background of the Invention

The vaccination of individuals to render the vaccinated individuals resistant to the development of infectious disease is one of the oldest forms of preventive care in medicine. Previously, vaccines for viral and bacterial pathogens for pediatric, adult, and veterinary usage were derived directly from the infectious organisms and could be categorized as falling into one of three broad categories: live attenuated, killed, and subunit vaccines. Although the three categories of vaccines differs significantly in their development and mode of actions, the administration of any of these three categories of these vaccines is intended to result in production of specific immunological response to the pathogen, following one or more inoculations of the vaccine. The resultive immunological response may or may not completely immunize the individual against subsequent infection, but will usually prevent the manifestation of disease symptoms and significantly limit the extent of any subsequent infection.

The techniques of modern molecular biology have enabled a variety of new vaccine strategies to be developed which are in various stages of pre-clinical and clinical development. The intent of these efforts is not only to produce new vaccines for old diseases, but also to yield new vaccines for infectious diseases in which classical vaccine development strategies have so far proven unsuccessful. Notably, the recent identification and spread of immunodeficiency viruses is an example of a pathogen for which classical vaccine development strategies have not yielded effective control to date.

The first broad category of classical vaccine is live attenuated vaccines. A live attenuated vaccine represents a specific strain of the pathogenic virus, or bacterium, which has been altered so as to lose its pathogenicity, but not its ability to infect and replicate in humans. Live attenuated vaccines are regarded as the most effective form of vaccine because they establish a true infection within the individual. The replicating pathogen and its infection of human cells stimulates both humoral and cellular compartments of the immune system as well as long lasting immunological memory. Thus, live attenuated vaccines for viral and intracellular bacterial infections stimulate the production of neutralizing antibodies as well as stimulating the production of cytotoxic t-lymphocytes (CTLs), usually after only a single inoculation. The ability of live attenuated vaccines to stimulate the production of CTLs is believed to be an important reason for the comparative effectiveness of live attenuated vaccines. CTLs are recognized as the main component of the immune system responsible for the actual clearing of viral and intracellular bacterial infections. CTLs are triggered by the production of foreign proteins in individual infected cells of the hosts, the infected cells processing the antigen and presenting the specific antigenic determinants on the cell surface for immunological recognition. The induction of CTL immunity by attenuated vaccines is due to the establishment of an actual, though limited, infection in the host cells including the production of foreign antigens in the individual infected cells. The vaccination process resulting from a live attenuated vaccine also results in the induction of immunological memory, which manifests itself in the prompt expansion of specific CTL clones and antibody-producing plasma cells in the event of future exposure to a pathogenic form of the infectious agent, resulting in the rapid clearing of this infection and practical protection from disease.

An important disadvantage of live attenuated vaccines is that they have an inherent tendency to revert to a new virulent phenotype through random genetic mutation. Although statistically such a reversion is a rare event for attenuated viral vaccines in common use today, such vaccines are administered on such a large scale that occasional reversions are inevitable, and documented cases of vaccine-induced illnesses exist. In addition, complications are sometimes observed when attenuated vaccines lead to the establishment of disseminated infections due to a lowered state of immune system competence in the vaccine recipient. Further limitations on the development of attenuated vaccines are that appropriate attenuated strains can be difficult to identify for some pathogens and that the frequency of mutagenic drift for some pathogens can be so great that the risk associated with reversion are simply unacceptable. A virus for which this latter point is particularly well exemplified is the human immunodeficiency virus (HIV) in which the lack of an appropriate animal model, as well as an incomplete understanding of its pathogenic mechanism, makes the identification and testing of attenuated mutant virus strains effectively impossible. Even if such mutants could be identified, the rapid rate of genetic drift and the tendency of retroviruses, such as HIV, to recombine would likely lead to an unacceptable level of instability in any attenuated phenotype of the virus. Due to these complications, the production of a live attenuated vaccine for certain viruses may be unacceptable, even though this approach efficiently produces the desired cytotoxic cellular immunity and immunological memory.

The second category of vaccines consists of killed and subunit vaccines. These vaccines consist of inactivated whole bacteria or viruses, or their purified components. These vaccines are derived from pathogenic viruses or bacteria which have been inactivated by physical or chemical processing, and either the whole microbial pathogen, or a purified component of the pathogen, is formulated as the vaccine. Vaccines of this category can be made relatively safe, through the inactivation procedure, but there is a trade-off between the extent of inactivation and the extent of the immune system reaction induced in the vaccinated patient. Too much inactivation can result in extensive changes in the conformation of immunological determinants such that subsequent immune responses to the product are not protective. This is best exemplified by clinical evaluation of inactivated measles and respiratory syncytil virus vaccines in the past, which resulted in strong antibody responses which not only failed to neutralize infectious virions, but exacerbated disease upon exposure to infectious virus. On the other extreme, if inactivated procedures are kept at a minimum to preserve immunogenicity, there is significant risk of incorporating infectious material in the vaccine formulation.

The main advantage of killed or subunit vaccines is that they can induce a significant titer of neutralizing antibodies in the vaccinated individual. Killed vaccines are generally more immunogenic than subunit vaccines, in that they elicit responses to multiple antigenic sites on the pathogen. Killed virus or subunit vaccines routinely require multiple inoculations to achieve the appropriate priming and booster responses, but the resultant immunity can be long lasting. These vaccines are particularly effective at preventing disease caused by toxin-producing bacteria, where the mode of protection is a significant titer of toxin neutralizing antibody. The antibody response can last for a significant period or rapidly rebound upon subsequent infection, due to an anamnestic or secondary response. On the other hand, these vaccines generally fail to produce a cytotoxic cellular immune response, making them less than ideal for preventing viral disease. Since cytotoxic lymphocytes are the primary vehicle for the elimination of viral infections, any vaccine strategy which cannot stimulate cytotoxic cellular immunity is usually the less preferred methodology for a virus disease, thereby resulting in attenuated virus being the usual methodology of choice.

The development of recombinant DNA technology has now made possible the heterologous production of any protein, of a microbial or viral pathogen, or part thereof, to be used as a vaccine. The vaccine constituents thus do not need to be derived from the actual pathogenic organism itself. In theory, for example, viral surface glycoproteins can be produced in eukaryotic expression systems in their native conformation for proper immunogenicity. However, in practice, recombinant viral protein constituents do not universally elicit protecting antibody responses. Further, as with killed vaccines, cellular cytotoxic immune responses are generally not seen after inoculation with a recombinant subunit protein. Thus, while this vaccine strategy offers an effective way of producing large quantities of a safe and potentially immunogenic viral or bacterial protein, such vaccines are capable of yielding only serum antibody responses and thus may not be the best choice for providing protection against viral disease.

The availability of recombinant DNA technology and the developments in immunology have led to the immunological fine mapping of the antigenic determinants of various microbial antigens. It is now theoretically possible, therefore, to develop chemically synthetic vaccines based on short peptides in which each peptide represents a distinct epitope or determinant. Progress has been made in identifying helper T-cell determinants, which are instrumental in driving B-cell or antibody immune responses. The covalent linkage of a helper T-cell peptide to a peptide representing a B-cell epitope, or antibody binding site, can dramatically increase the immunogenicity of the B-cell epitope. Unfortunately, many natural antibody binding sites on viruses are conformationally dependent, or are composed of more than one peptide chain, such that the structure of the epitope on the intact virus becomes difficult to mimic with a synthetic peptide. Thus peptide vaccines do not appear to be the best vehicle for the stimulation of neutralizing antibodies for viral pathogens. On the other hand, there is some preliminary evidence that peptides representing the determinants recognized by cytotoxic T-lymphocytes can induce CTLs, if they are targeted to the membranes of cells bearing class I major histocompatibility (MHC) antigens, via coupling to a lipophilic moiety. These experimental peptide vaccines appear safe and inexpensive, but have some difficulty in mimicking complex three dimensional protein structures, although there is some evidence that they can be coaxed into eliciting cytotoxic immunity in experimental animals.

Another new recombinant technique which has been proposed for vaccines is to create live recombinant vaccines representing non-pathogenic viruses, such as a vaccinia virus or adenovirus, in which a segment of the viral genome has been replaced with a gene encoding a viral antigen from a heterologous, pathogenic virus. Research has indicated that infection of experimental animals with such a recombinant virus leads to the production of a variety of viral proteins, including the heterologous protein. The end result is usually a cytotoxic cellular immune response to the heterologous protein caused by its production after inoculation. Often a detectable antibody response is seen as well. Live recombinant viruses are, therefore, similar to attenuated viruses in their mode of action and result in immune responses, but do not exhibit the tendency to revert to a more virulent phenotype. On the other hand, the strategy is not without disadvantage in that vaccinia virus and adenovirus, though non-pathogenic, can still induce pathogenic infections at a low frequency. Thus it would not be indicated for use with immune-compromised individuals, due to the possibility of a catastrophic disseminated infection. In addition, the ability of these vaccines to induce immunity to a heterologous protein may be compromised by pre-existing immunity to the carrier virus, thus preventing a successful infection with the recombinant virus, and thereby preventing production of the heterologous protein.

In summary, all of the vaccine strategies described above possess unique advantages and disadvantages which limit their usefulness against various infectious agents. Several strategies employ non-replicating antigens. While these strategies can be used for the induction of serum antibodies which may be neutralizing, such vaccines require multiple inoculations and do not produce cytotoxic immunity. For viral disease, attenuated viruses are regarded as the most effective, due to their ability to produce potent cytotoxic immunity and lasting immunological memory. However, safe attenuated vaccines cannot be developed for all viral pathogens.

It is therefore desirable that vaccines be developed which are capable of producing cytotoxic immunity, immunological memory as well as the production of circulating antibodies, without having any unacceptable risk of pathogenicity, or mutation, or recombination of the virus in the vaccinated individual.

### Summary of the Invention

The present invention is summarized in that a genetic vaccine construction derived from the human immunodeficiency virus (HIV) is used to manufacture a medicament for the vaccination of an animal against HIV by a genetic vaccination method including the steps of preparing copies of a foreign genetic construction including a promoter operative in cells of the animal and a protein coding region coding for a determinant produced by the virus, coating the copies of the foreign genetic construction onto carrier particles small in size in relation to the size of the cells of the animal, and accelerating the coated carrier particles into cells of the animal in vivo.

The present invention is also summarized in that a genetic vaccine for HIV. is created by joining a DNA sequence encoding all of the open reading frames of the viral genome, but not the long terminal repeats or primer binding site, to a promoter effective in human cells to make a genetic vaccine and then transducing the genetic vaccine into cells of an individual by a particle mediated transfection process.

It is a feature of the present invention in that it is equally adopted to either epidermal or mucosal delivery of the genetic vaccine or delivery into peripheral blood cells, and thus may be used to induce a serum as well as a mucosal antibody response in the treated individual.

It is an advantage of the genetic vaccination method of the present invention in that it is inherently safe, is not painful to administer, and should not result in adverse consequences to vaccinated individuals.

Other objects, advantages and features of the present invention will become apparent from the following specification.

### Brief Description of the Drawings

Fig. 1 is a plasmid map, showing genes and restriction sites, of the plasmid pWRG1602.
Fig. 2 is a plasmid map of the genetic vaccine plasmid pCHIVpAL.
Fig. 3 is a graphical illustration of some of the results from one of the examples below.
Fig. 4 is a graphical illustration of the results from another of the examples below.

### Description of the Preferred Embodiment

The present invention is intended to create genetic vaccines for the immunodeficiency viruses, by transfecting somatic cells in the animal to be immunized with a gene sequence capable of causing expression in cells of the animal the antigenically correct proteins from the pathogenic virus, the gene sequence not including elements of the viral genome necessary for viral replication or pathogenesis. The introduction of such a genetic vaccine construction into animal somatic cells is intended to mimic the actions of attenuated or live recombinant viruses without the risk of pathogenic infection in either healthy or immune-compromised individuals. The introduction of the genetic vaccine will cause to be expressed within those cells the structural protein determinants associated with the virus particle. The processed structural proteins will be displayed on the cellular surface of the transfected cells in conjunction with the major histocompatibility complex (MHC) antigens of the normal cell. The display of these antigenic virus determinants in association with the MHC antigens is intended to elicit the proliferation of cytotoxic T-cell clones specific to the viral determinants. Furthermore, the structural proteins released by the expressing transfected cells can also be picked up by antigen presenting cells to trigger antibody responses. The genetic vaccine construction capable of eliciting this immune response is preferably delivered into the transfected cells by means of an accelerated-particle transformation system.

For several reasons, the genetic vaccine approach of the present invention is particularly advantageously used for vaccination against immunodeficiency viruses, such as human immunodeficiency virus (HIV) and related animal viruses, simian immunodeficiency virus (SIV) and feline immunodeficiency virus (FIV). The HIV virus does not lend itself to attenuated vaccine approaches due to the inherent possibility of reversion of mutated forms of this virus. While viral protein subunit vaccines for these viruses are under development, such subunit vaccines cannot produce a cytotoxic response, which may be necessary to prevent the establishment of HIV infection or HIV-related disease. The use of a genetic vaccine transfection strategy as described here would trigger a cytotoxic response. Also this vaccine approach allows for delivery to mucosal tissues which may aid in conferring resistance to viral introduction, which with HIV is believed to sometimes occur through mucosal membranes.

In order to achieve the cellular immune response sought in the vaccination process of the present invention, a genetic vaccine construction must be created which is capable of causing transfected cells of the vaccinated individual to express the major antigenic determinants from the virus. This can be done by identifying the coding regions for the various proteins which are encoded by the genome of the virus, creating a synthetic coding sequence for each such protein, and joining each of such coding sequences to promoters capable of expressing the sequences in mammalian cells. Alternatively, the viral genome itself can be used. For a retrovirus, the coding sequence can be made from the DNA form of the viral genome, the provirus, as long as the provirus clone has been altered so as to remove from it the sequences necessary for viral replication in infectious processes such as the long terminal repeats and the primer binding site. Such a provirus clone would inherently have the mRNA processing sequences necessary to cause expression of all of the viral structural proteins in transfected cells.

The viral genetic material must be altered to prevent the pathogenic process from beginning. The method of altering the virus will vary from virus to virus. The immunodeficiency virus is a retrovirus carrying its genetic material in the form of RNA. During the normal infection process, the RNA is processed by an enzyme, referred to as reverse transcriptase, which converts the RNA sequence of the virus into a DNA form, known as the provirus. The provirus for the human immunodeficiency virus (HIV) has as many as a dozen open reading frames, all of which are translated to produce proteins during the infectious process. Some of the proteins are structural, and others are regulatory for steps in the infectious process. As it happens, all of the proteins produced from the provirus are actually produced from a single mRNA precursor which is differentially spliced to produce a variety of differently spliced RNA products, which are translated into the various proteins expressed by the virus. Advantageously, it would be helpful if the transfected cell utilized in the vaccination process of the present invention expressed as many of the viral structural proteins as possible. Accordingly, it would be desirable to use as much as possible of the provirus as the genetic vaccine coding sequence for this genetic vaccine, assuming only that sufficient portions are removed from the provirus so as to render it incapable of initiating a viral replication stage in a vaccinated individual.

A convenient strategy for achieving this objective with either the HIV or SIV viruses is based on the fact that the infectious viruses have important genetic elements necessary for replication of the viral genome, known as the long terminal repeat (LTR) elements and the primer binding site, and which are located at the ends of the native provirus sequence. The primer binding site is the site on the viral RNA where a tRNA recognizes the viral RNA, and binds to it to serve as a primer for the initiation of the reverse transcription process. Both the LTR elements and the primer binding site are necessary to permit reverse transcription to occur. Removing either the LTRs or the primer binding site would impede viral replication. Removing both the LTRs and the primer binding site from the DNA provirus ensures that the genetic sequence thus created is incapable of causing viral replication or the encoding of pathogenic viral particles.

Thus, the use of intact viral coding regions for structural and regulatory proteins is preferred for the present invention, both for reasons of convenient genetic vaccine assembly and to ensure the correct production of as many viral proteins as possible to effectively mimic a natural infection. However, in some instances it may be desirable and sufficient to express only one or a few viral proteins without producing the whole set of viral proteins. This can be done by assembling an individual expression vector for each desired viral protein using standard recombinant techniques.

To properly express the viral genetic sequence in transfected cells, a promoter sequence operable in the target cells is needed. Several such promoters are known for mammalian systems which may be joined 5', or upstream, of the coding sequence for the protein to be expressed. A downstream transcriptional terminator, or polyadenylation sequence, may also be added 3' to the protein coding sequence.

In order to be effective as a genetic vaccine, a method is required to deliver the genetic sequence encoding the viral proteins into the cells of the individual sought to be immunized. There are several techniques by which genes can be delivered into the somatic cells of mammals subject to immunodeficiency viral disease. Known techniques include direct DNA injection, use of retroviral vectors to deliver genetic sequences into somatic cells, or the delivery of DNA in liposomes or other encapsulation agents which can direct the delivery of the encapsulated DNA into one or more cell types of a susceptible individual. However, it is preferred for the present invention that the DNA be transferred into the susceptible individual by means of an accelerated particle gene transfer device. The technique of accelerated-particle gene delivery is based on the coating of genetic constructions to be delivered into cells onto extremely small carrier particles, which are designed to be small in relation to the cells sought to be transformed by the process. The coated carrier particles are then physically accelerated toward the cells to be transformed such that the carrier particles lodge in the interior of the target cells. This technique can be used either with cells in vitro or in vivo. At some frequency, the DNA which has been previously coated onto the carrier particles is expressed in the target cells. This gene expression technique has been demonstrated to work in procaryotes and eukaryotes, from bacteria and yeasts to higher plants and animals. Thus, the accelerated particle method provides a convenient methodology for delivering genes into the cells of a wide variety of tissue types, and offers the capability of delivering those genes to cells in situ and in vivo without any adverse impact or effect on the treated individual.

The general approach of accelerated particle gene transformation technology is described in U.S. Patent No. 4,945,050 to Sanford. An instrument based on an improved variant of that approach is available commercially from Biorad Laboratories. An alternative approach to an accelerated particle transformation apparatus is disclosed in U.S. Patent No. 5,015,580 which, while directed to the transformation of soybean plants, describes an apparatus which is equally adaptable for use with mammalian cells and intact whole mammals.

It is also specifically envisioned that aqueous droplets containing naked DNA, including the viral genetic vaccine therein, can be delivered by suitable acceleration techniques into the tissues of the individual sought to be vaccinated. At some frequence, such "naked" DNA will be taken up in the treated tissues.

The term transfected is used herein to refer to cells which have incorporated the delivered foreign genetic vaccine construction, whichever delivery technique is used. The term transfection is used in preference to the term transformation to avoid the ambiguity inherent in the latter term, which is also used to refer to cellular changes in the process of oncogenesis.

With the accelerated particle device , a genetic vaccine can be delivered in a non-invasive manner to a variety of susceptible tissue types in order to achieve the desired antigenic response in the individual. Most conveniently, the genetic vaccine can be introduced into the epidermis. Such delivery it has been found, will produce a systemic humoral immune response and will likely also stimulate a cytotoxic immune response. To obtain additional effectiveness from this technique, it may also be desirable that the genes be delivered to a mucosal tissue surface, in order to ensure that a mucosal as well as a systemic antigenic response is produced in the vaccinated individual. It is envisioned that there are a variety of suitable delivery sites available including any number of sites on the epidermis, peripheral blood cells, i.e. lymphocytes, which could be treated in vitro and placed back into the individual, and a variety of oral, upper respiratory, and genetal mucosal surfaces.

The adequacy of the immunodeficiency virus vaccine expression vectors to be transfected into cells by accelerated particle transfection processes can be assessed by assaying for viral antigenic production in mammalian cells in vitro. Susceptible mammalian cells of a cell type which can be maintained in culture, such as monkey COS cells, can be transfected in vitro by any of a number of cell transfection techniques, including calcium phosphate-mediated transfection, as well as accelerated particle transfection. Once the genetic vaccine expression vector is introduced into the susceptible cells, the expression of the viral antigens can then be monitored in medium supernatants of the culture of such cells by a variety of techniques including ELISA, Western blotting, or reverse transcriptase assay.

After confirmation that a given expression vector is effective in inducing the appropriate viral protein production in cultured cells in vitro, it can then be demonstrated that such a vector serves to induce similar protein production in a small animal model such as the mouse. The measurement of antibody and cytotoxic cellular immune responses in mice in response to such a genetic vaccine would be an important demonstration of the concept and justify the initiation of more rigorous testing in an appropriate animal challenge model.

Once it is determined that the genetic vaccine is capable of inducing viral protein production and immune responses in small laboratory animals, it then becomes necessary to determine the dosage and timing suitable to produce meaningful immune responses in an animal model for human immunodeficiency disease. This can be done most effectively using the SIV and rhesus macaque model. Animals would receive several doses of the expression constructs by accelerated particle transfection techniques at a variety of tissues sites. The treated tissue sites would include, but would not be limited to, the epidermis, dermis (through the epidermis), the oral cavity and upper respiratory mucosa, and peripheral blood cells. Various challenge techniques would be utilized, and the number and timing of doses of a genetic vaccine would be systematically varied in order to optimize any resulting immunogenic response, and to determine which dosage routines resulted in maximum response. Antibody responses in the treated individuals can be detected by any of the known techniques for recognizing antibodies to specific viral antigens, again using standard Western blot and ELISA techniques. It is also possible to detect the cell mediated cytotoxic response, using standard methodologies known to those of ordinary skill in immunological biology. Specifically, the presence of cytotoxic T-cells in the spleen or peripheral blood can be indicated by the presence of lytic activity, which recognizes histocompatible target cells which are themselves expressing the viral antigens from the immunodeficiency virus.

While the best tissue sites for the delivery of a genetic vaccine for human immunodeficiency disease and the number and timing of doses must be empirically determined in an animal model and later confirmed in clinical studies, it is difficult at this point to predict the precise manner in which such a vaccine would be used in an actual human health care setting. Because such a vaccine is intended to mimic the effects of a live attenuated or recombinant viral vaccine without any risks of pathogenicity, it is conceivable that such a vaccine would not only be useful in the induction of protective immunity in naive individuals, but would also be indicated for use in HIV-infected, immune compromised individuals for the purpose of artificially boosting their immune status to forestall or impede the progression of disease. It is also important to consider that no single vaccine strategy may in itself be capable of inducing the variety of immunological responses necessary to either achieve prophylaxis in healthy individuals or forestall progression of disease in infected patients. Rather, a combination of approaches may demonstrate a true synergy in achieving these goals. Thus, it is conceivable that a combined vaccine approach incorporating a genetic vaccine, which mimics a true infection, and a killed or subunit vaccine would be an attractive way to efficiently achieve cytotoxic immunity and immunological memory as well as high levels of protective antibody. Genetic vaccines should serve as a safe alternative to the use of live vaccines and could be used in a variety of immunization protocols and in combination with other vaccines to achieve the desired results.

### EXAMPLES

### 1. Construction of Vectors

The genetic sequences for the human immunodeficiency virus (HIV) and simian immunodeficiency virus (SIV) have been fully determined, published, and are generally available. For example, the DNA sequence for the HIV strain designated LAV-1/BRU is found in GenBank at Accession Number K02013, and the nucleotide positions referred to below are from that sequence. Samples of both HIV and SIV are readily available to qualified experimenters through appropriate depositories in health research facilities.

The HIV genetic vaccine expression vector was constructed to include an 8266 base pair fragment derived from the proviral genome of HIV strain LAV-1/BRU. The fragment was the portion of the HIV DNA provirus sequence beginning at the Sac I site in nucleotide position 678 and ending at the Xho I site at nucleotide number 8944 (the nucleotide numbering convention used here assumes that nucleotide number 1 corresponds to the first nucleotide of the U3 region of the 5' LTR). This designated fragment of the HIV genome contains all of the viral open reading frames, excepting only for a portion which encodes the carboxyl terminus of the nef protein. This fragment, once transcribed, results in an mRNA which contains all of the splicing donor and acceptor sites necessary to effectuate the RNA splicing pathways actuated in an infected cell during the pathogenic process initiated by the HIV virus.

This coding sequence fragment must be coupled to a promoter capable of expression in mammalian cells in order to achieve expression of the viral antigenic proteins in a susceptible cell. Once coupled to a promoter, this coding sequence fragment leads to the expression of the major open reading frames from the virus (including gag, pol, and env) and makes use of the native ribosomal frame shifting and mRNA processing pathways, in the same fashion as would be utilized by the virus itself. However, this fragment does lack certain viral genetic elements necessary for replication of the viral genome, including specifically the long terminal repeat (LTR) elements and the primer binding site. Thus the fragment is incapable of reverse transcription, thus inhibiting any potential pathogenic process from occurring with this genetic sequence.

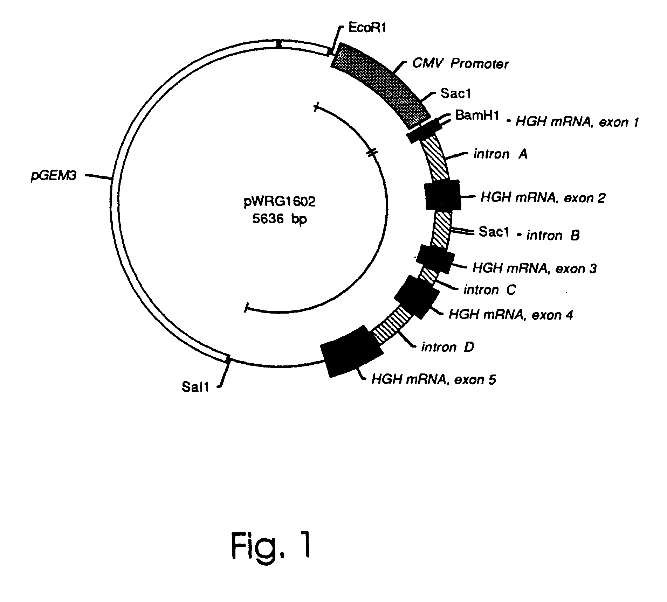

To couple the coding sequence encoding the HIV antigens to a promoter capable of expression in mammalian cells, the human cytomegalovirus (HCMV) immediate early promoter was used. An expression cassette had been constructed, designated pWRG1602, which is illustrated by the plasmid map of Figure 1. The plasmid pWRG1602 yields a 660 base pair Eco R1 and Bam H1 restriction fragment which contains several synthetic restriction sites which had been added to the end of a 619 base pair region containing the HCMV immediate early promoter.

The transcription termination segment utilized was a polyadenylation sequence from the SV40 virus. The SV40 polyadenylation fragment is an approximately 800 base pair fragment (obtained by Bgl II and Bam HI digestion) derived from the plasmid psv2dhfr, which was formerly commercially available from the Bethesda Research Labs, catalog number 5369SS. The same polyadenylation fragment is also described in Subramani, et al., Mol. Cell. Biol., 1:854-864 (1981). This fragment also contains a small SV40 intervening sequence near the Bgl II end, with the SV40 polyadenylation region lying toward the Bam HI end of the fragment.

An HIV genetic vaccine expression plasmid, designated pCHIVpAL, was constructed in the following manner. The 800 base pair SV40 fragment from psv2dhfr was treated with Klenow DNA polymerase to "fill in" the overhanging termini. In parallel, a quantity of Bluescript M13+SK DNA cleaved with Xho I (Accession Number X52325, with the Xho I site at position 668) and similarly treated with Klenow DNA polymerase. The two fragments were ligated resulting in a plasmid designated pBSpAL. The orientation of the SV40 fragment in the Bluescript vector was such that the Bam HI end, or the end containing the polyadenylation site, was oriented toward the main body of the polylinker contained in the plasmid.

Quantities of the plasmid pBSpAL were then digested with the restriction enzymes Sac I and Sal I to create a plasmid having compatible ends for ligation to a fragment created by Xho I and Sac I digestion. To this plasmid was ligated the 8266 base pair fragment from the HIV provirus, resulting in a plasmid designated pHIVpAL, which now contains the HIV antigenic determinants coding region followed by the SV40 polyadenylation signal.

Then the plasmid pHIVpAL was cleaved with the restriction enzyme Sac I, and the 3' overhanging ends were deleted using Klenow DNA polymerase. Into the opening thus created, the 660 base pair fragment containing the HCMV promoter (the ends of which had been filled with Klenow DNA polymerase) was inserted.

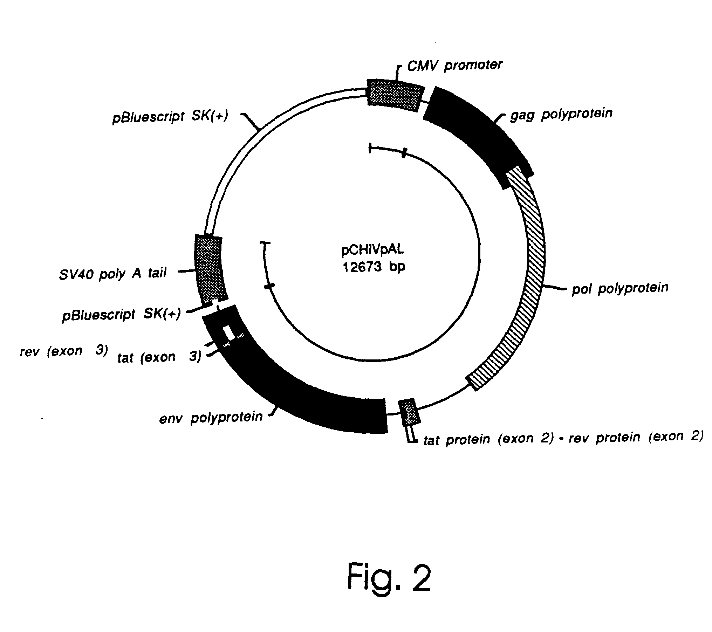

The result is the plasmid designated in Figure 2 which contains, oriented 5' to 3', the HCMV immediate early promoter, the 8266 base pair fragment from the HIV genome encoding all the important open reading frames on the virus. and the SV40 polyadenvlation fragment. This construct served as a genetic vaccine construction for the method of the present invention.

The SIV expression vector was constructed in a manner analogous to the HIV expression vector except utilizing, in lieu of the HIV gene sequence, an 8404 base pair fragment from the proviral genome of SIVmac239 (found in GenBank at Accession No. M33262), beginning at the Nar I site at nucleotide position 823 and ending at the Sac I site at nucleotide position 9226 (following the same nucleotide numbering convention as with the HIV). The SIV genome expression fragment can be substituted for the HIV genome fragment plasmid pCHIVpAL above.

### 2. Introduction of Genetic Vaccine into Cells in Culture

To verify the ability of the genetic construct to express the proper antigenic proteins in mammalian cells, an in vitro test was conducted.

Quantities of the plasmid pCHIVpAL were reproduced in vitro. Copies of the DNA of this plasmid were then coated onto gold carrier particles before transfection into cells in culture. This was done by mixing 10 milligrams of precipitated gold powder (1.5 micron average diameter) with 50 microliters of 0.1 M spermidine and 25 micrograms of DNA of the plasmid pCHIVpAL. The mixture was incubated at room temperature for 10 minutes. Then, 50 microliters of the 2.5 M CaCl₂ was added to the mixture, while continuously agitating, after which the sample was incubated an additional 3 minutes at room temperature to permit precipitation of the DNA onto the carrier particles. The mixture was centrifuged for 30 seconds in a microcentrifuge to concentrate the carrier particles with the DNA thereon, after which the carrier particles were washed gently with ethanol and resuspended in 10 milliliters of ethanol in a glass capped vial. The resuspension of the carrier particles in the ethanol was aided by immersion of the vial in a sonicating water bath for several seconds.

The DNA-coated carrier particles were then layered onto 35 millimeter square mylar sheets (1.7 cm on each side) at a rate of 170 microliters of DNA-coated gold carrier particles per mylar sheet. This was done by applying the ethanol suspension of the carrier particles onto the carrier sheet and then allowing the ethanol to evaporate. The DNA-coated gold particles on each mylar sheet were then placed in an accelerated particle transformation apparatus of the type described in U.S. Patent No. 5,015,580, which utilizes an adjustable electric spark discharge to accelerate the carrier particle at the target cells to be transfected by the carrier DNA.

Meanwhile, a culture of monkey COS-7 cells had been prepared in a 3.5 cm culture dish. The medium was temporarily removed from the COS cells, and the culture dish was inverted to serve as the target surface for the accelerated particle transfection process. A spark discharge of 8 kilovolts was utilized in the process described in more detail in the above-identified U.S. Patent No. 5,015,580. After the particle injection into the cells, two milliliters of fresh medium was added to the culture dish to facilitate continued viability of the cells.

Twenty-four hours following the accelerated particle delivery, the medium was harvested from 35 culture dishes of the cells and concentrated to test for high molecular weight HIV antigens. The medium was concentrated by high speed centrifugation. 0.5 milliliters of the unconcentrated medium was set aside for future determination of HIV p24 content, using a commercial ELISA kit. Fresh growth medium was added to the plates to allow continued monitoring of HIV antigen production. The harvested medium was cleared of cellular debris by first centrifuging it 1500 RPM in a standard laboratory centrifuge, and then filtering it through a 0.45 micron membrane. The cleared filtrate was layered gently onto 1 milliliter cushions of 20% glycerol, 100 mM KCl, 50 mM Tris-HCl, pH 7.8 in each of 6 ultracentrifuge tubes (Beckman SW41 rotor). The samples were centrifuged at 35,000 RPM for 70 minutes at 4°C. Following centrifugation, the medium was discarded, and the pellets were resuspended in a total of 20 microliters as 0.15 M NaCl, 50 mM Tris-HCl, pH 7.5, 1 mM EDTA.

The concentrated sample was subjected to an electrophoresis process in a pre-cast 12% SDS-polyacrylamide gel (Bio-Rad). The gel was electroblotted onto a nitrocellulose sheet using a Buchler semi-dry blotter (Model Number 433-2900) according to the manufacturer's directions. Assays were then conducted to detect the HIV viral antigens immobilized on the nitrocellulose sheet. The HIV antigens p24 and gp120 were detected using a Bio-Rad immunoblot assay kit (Catalog number 170-6451). To utilize the immunoblot assay kit, specific antibodies for the antigens sought to be detected are required. For use in the HIV specific assay, the following monoclonal antibodies, which are commercially available, were used. For gp120, the monoclonal antibodies number 1001 from American Bio-Technologies and NEA 9305 from DuPont. For the antigen p24, monoclonal antibody number 4001 from American Bio-Technologies and antibody NEA 9283 from DuPont was utilized. The immunoblot assay was performed according to the manufacturer's directions, except for the direct substitution of Carnation non-fat dry milk for gelatin in all solutions calling for gelatin. The developed immunoblot assay revealed bands corresponding to both the gp120 and p24 antigens produced in the sample from the treated COS cells. A negative control produced no such bands and positive controls consisting of the antigenic proteins themselves produced bands similar to those from the samples from the treated cells. This confirmed the activity, and expression, of the plasmid pCHIVpAL in the COS cells, and also confirmed that the molecular weight forms of the antigens were similar to those produced in the normal host cells for the virus. A parallel sample derived from non-treated COS cells showed no evidence of reactivity. There was a slight difference in mobility between the gp120 band derived from the COS cells, and the gp120 band in the positive control, which was believed to be due to differences in glycosylation.

To further demonstrate the production of HIV determinants in monkey COS cells, growth medium from treated cells was analyzed using a Coulter HIVp24 antigen assay kit (Catalog number 6603698). Samples of growth medium from the first three 24 hour periods following gene delivery were assayed for p24 antigen content, and showed to contain 42, 30, and 12 nanograms per milliliter respectively of the p24 antigen. These values reflect the amount of p24 antigen released into the medium during each 24 hour period, since the growth medium for the cells was changed completely each day following treatment. Parallel samples from non-treated COS cells exhibited no reactivity.

### 3. Detection of Viral Antigen in the Skin of Intact Mice

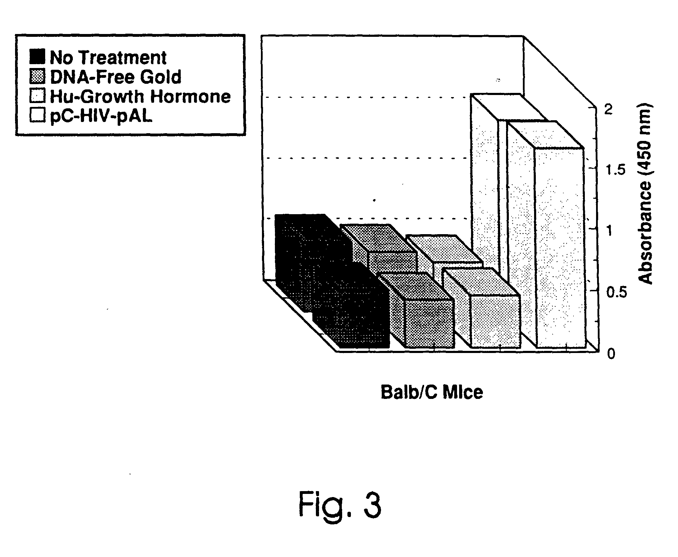

An accelerated particle transfection protocol was then used to deliver the plasmid pCHIVpAL into the skin of intact whole mice. It has previously been demonstrated that accelerated particles may be utilized to deliver genes into the epidermis or dermal layer of intact animals and that the genes will express once delivered. Copies of the plasmid pCHIVpAL were coated onto gold carrier particles, as described in the prior example, except that five micrograms of the plasmid was used per milligram of the gold carrier particles and the preparation was suspended in ethanol at a concentration of 5 mg of carrier particle per ml of ethanol. One hundred sixty-three microliters of this suspension was loaded onto each of two carrier sheets, for use in a particle acceleration protocol into intact whole mice. Two additional suspensions of the gold carrier particles were prepared as controls. The first control preparation utilized a plasmid containing the human growth hormone gene, and the second was prepared without the addition of any DNA onto the gold carrier particles. Six Balb/C mice were anesthetized with 50 microliters of a 10:2 mixture of Ketamine/Rompin, and the abdominal hairs of the mice were shaved with clippers. Hair follicles were removed with a depilatory cream (Nair). The anesthetized mice were suspended 15 millimeters above the retaining screen on a particle delivery chamber using a plastic petri dish as a spacer, using the method described in published PCT application No. WO 91/19781. A square hole was cut in the bottom of the petri dish to allow the accelerated carrier particles to access the abdominal skin layer of the anesthetized mice. The six mice were divided into three sets of two mice each. The mice in each of the three sets received a single "blast" of carrier particles, which were accelerated utilizing an electric discharge voltage of 25 kV. The mice in each of the three sets received treatments representing the pCHIVpAL, the growth hormone plasmid, or the gold carrier particles free of DNA, respectively. Three days following treatment, the target skin areas were excised from the treated mice, as well as from the two control mice which had not been subjected to any particle-mediated transfection protocols. The tissue samples were minced with dissecting scissors in 600 microliters of phosphate buffered saline containing 0.5% Triton X-100. The tissue suspensions were then centrifuged at 5,000 RPM for five minutes and the resulting supernatants were collected. The supernatant samples were diluted ten-fold and analyzed for HIV p24 antigen content using the Coulter HIV p24 antigen ELISA kit (catalog number 6603698) utilizing the directions of the manufacturer. Figure 3 illustrates the results of this protocol. Tissue samples from the pCHIVpAL treated mice exhibited 3-fold more reactivity than the control samples, indicating that the treated tissues were synthesizing HIV p24 antigen as a result of the gene delivery protocol. After subtraction of background, this level of reactivity is consistent with the release of 0.6 nanograms of HIV p24 antigen from the minced tissue when compared to a standard curve generated with positive control reagents in the ELISA kit.

### 4. Detection of Serum IgG Antibodies Specific to HIV p24 in Vaccinated Mice

The next experiment was conducted to test the ability of mice to exhibit a systemic immune response to foreign proteins expressed as a result of gene delivery into epidermal cells of the mice. Copies of the plasmid pCHIVpAL were created and coated onto gold carrier particles as described in Examples 1 and 2 above, except that 10 micrograms of plasmid DNA was used per milligram of the gold carrier particles. As a control, a heterologous plasmid containing the human growth hormone gene was also used for preparing plasmid-coated gold carrier particles for in vivo gene delivery. For this example, 5.0 micrograms of DNA was used per milligram of gold carrier particles due, to the smaller size of human growth hormone plasmid, and so as to have approximately the same number of copies of the plasmid delivered to the cells in vivo.

Ten male Balb/C mice (5 to 7 weeks old) were divided into three groups of four, three, and three mice, respectively. A first step of priming immunization was conducted on the four mice in group 1, in which each received a single treatment of accelerated particles coated only with the growth hormone plasmid. The acceleration was conducted at 25 kV by the method as described in Example 3 above. The three mice in group 2 each received a single treatment of gold carrier particles coated with the plasmid pCHIVpAL. The mice in group 3 each received three abdominal treatments of accelerated particles which were coated with pCHIVpAL. In the case of group 3, the blast areas were arranged so as not to be overlapping. The blasting routine for all three of the groups was repeated four and seven weeks later in order to boost the immune responses. Eight to ten days following the last treatment, retro-orbital blood samples were taken from each mouse and allowed to coagulate at 4°C in microtainer tubes. Following centrifugation at 5000 RPM, the serum was collected.

An assay was next conducted to detect HIV p24-specific antibodies in the mouse serum by an enzyme immunoassay. This assay was performed by adsorbing 0.4 micrograms of recombinant HIV p24 antigen (American Bio-Technologies, Inc.) to each well of a 96 well microtiter plate in 50 microliter Dulbecco's phosphate buffered saline (D-PBS) by incubating overnight at 4°C. Following adsorbtion of the antigen, the remaining protein binding sites were blocked by the addition of D-PBS containing 2% Carnation non-fat dry milk (200 microliters per well) for two hours. The wells were then washed three times with 300 microliters D-PBS containing 0.025% Tween-20. The serum samples of 5 microliters each were diluted 1:10 with D-PBS (45 microliters), and added to a single well following which they were incubated at room temperature for one hour. After washing with D-PBS Tween-20 as described above, the presence of bound mouse antibody was detected using a goat-anti-mouse alkaline phosphatase conjugated second antibody (Bio-Rad, catalog number 172-1015) diluted 1:1500 in D-PBS-Tween-20 (50 microliters per well). After incubation for 30 minutes at room temperature, the wells were washed again and the conjugated antibody was detected using a Bio-Rad alkaline phosphatase substrate kit (Catalog No. 172-1063) according to the manufacturer's instructions. The ELISA plate was analyzed on a microplate reader using a 405 nm filter.

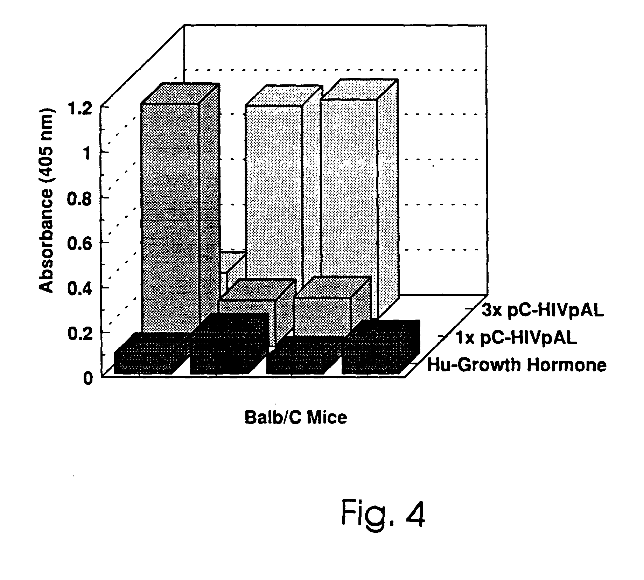

The results of this assay are illustrated in Figure 4. One of the mice from the group which received single blasts of the pCHIVpAL coated gold particles and two mice from the group which received three blasts of the same particles exhibited significant p24-specific antibody responses (5 to 10 fold above background). All of the sera from the control animals exhibited typical background ELISA reactivity. This example demonstrates the feasibility of inducing antigen-specific antibody responses following epidermal delivery of antigen-encoded genes coated on carrier particles into cells in an intact animal in vivo.

Thus it is demonstrated that circulating levels of antibodies to an immunodeficiency virus antigens can be created in vivo by delivering into the patient not quantities of the antigenic proteins of the virus, or the virus itself, but rather by instead delivering into the patient to be treated gene sequences causing expression of the antigenic proteins in cells in the vaccinated individual. This method thus enables the creation of a serum antibody response in vaccinated individuals without the necessity for delivering into the individual either any portions of live virus or any portions of the genetic material which are capable of effectuating replication of the virus in individuals.

## Claims

1. Use of a genetic vaccine construction for the manufacture of a medicament for inducing an immunological response to an immunodeficiency virus in a primate, wherein:
- the genetic vaccine construction includes a promoter operative in cells of the primate and a protein coding region encoding antigenic determinants of said virus but does not include sequences essential for replication of the virus;
- the genetic vaccine construction is coated onto carrier particles small in size in relation to the size of the cells of the primate, thereby to manufacture said medicament, and
- said immunological response is induced by accelerating the coated carrier particles into the skin of the primate.

2. Use according to claim 1, wherein the virus is human immunodeficiency virus.

3. Use according to claim 2, wherein the promoter is the human cytomegalovirus immediate early promoter.

4. Use according to any one of the preceding claims, wherein the protein coding region encodes the major antigenic determinants of the virus.

5. Use according to any one of the preceding claims, wherein the protein coding region includes DNA sufficient to code for all of the determinants encoded by the viral genome.

6. Use according to any one of the preceding claims, wherein the sequences essential for replication are the long terminal repeat elements and the primer binding site.

7. Use according to any one of the preceding claims, wherein the carrier particles are gold particles.

8. An accelerated particle gene transfer device for inducing an immunological response to an immunodeficiency virus in a primate, said device being loaded with carrier particles that are small in size in relation to the size of cells of the primate and that are coated with a genetic vaccine construction including a promoter operative in said cells and a protein coding region encoding antigenic determinants of said virus but not including sequences essential for replication of the virus.

9. A device according to claim 8, wherein the virus is human immunodeficiency virus.

10. A device according to claim 9, wherein the promoter is the human cytomegalovirus immediate early promoter.

11. A device according to any one of claims 8 to 10, wherein the protein coding region encodes the major antigenic determinants of the virus.

12. A device according to any one of claims 8 to 11, wherein the protein coding region includes DNA sufficient to code for all of the determinants encoded by the viral genome.

13. A device according to any one of claims 8 to 12 wherein the sequences essential for replication are the long terminal repeat elements and the primer binding site.

14. A device according to any one of claims 8 to 13, wherein the carrier particles are gold particles.

## Patentansprüche

1. Anwendung einer genetischen Vakzine-Konstruktion für die Herstellung eines Medikaments zum Induzieren einer immunologischen Antwort auf ein Immunschwäche-Virus in einem Primaten, wobei:
- die genetische Vakzine-Konstruktion einen in Zellen des Primaten operativen Promotor und eine Protein-codierende Region umfasst, die antigene Determinanten des Virus codiert, doch keine für die Replikation des Virus essentiellen Sequenzen enthält;
- die genetische Vakzine-Konstruktion auf Trägerpartikel aufgeschichtet ist, die von kleiner Größe relativ zur Größe der Zellen des Primaten sind, um dadurch das Medikament herzustellen; und
- die immunologische Antwort durch Akzelerieren der beschichteten Trägerpartikel in die Haut des Primaten induziert wird.

2. Anwendung nach Anspruch 1, wobei das Virus ein humanes Immunschwäche-Virus ist.

3. Anwendung nach Anspruch 2, wobei der Promotor der Immediate-Early-Promotor des humanen Cytomegalovirus ist.

4. Anwendung nach einem der vorangehenden Ansprüche, wobei die Protein-codierende Region die antigenen Haupt-Determinanten des Virus codiert.

5. Anwendung nach einem der vorangehenden Ansprüche, wobei die Protein-codierende Region ausreichend DNA enthält, um für alle der Determinanten zu codieren, die durch das viraleGenom codiert sind.

6. Verwendung nach einem der vorangehenden Ansprüche, wobei die für die Replikation essentiellen Sequenzen die Long Terminal Repeat-Elemente und die Primer-Bindungsstelle sind.

7. Verwendung nach einem der vorangehenden Ansprüche, wobei die Trägerpartikel Goldpartikel sind.

8. Gentransferelement für akzelerierte Partikel zum Induzieren einer immunologischen Antwort auf ein Immunschwäche-Virus in einem Primaten, welche Vorrichtung mit Trägerpartikeln beladen ist, die von kleiner Größe relativ zur Größe der Zellen des Primaten sind und die mit einer genetischen Vakzine-Konstruktion beschichtet sind, einschließlich eines in diesen Zellen operativen Promotors und einer Protein-codierenden Region, die antigene Determinanten des Virus codiert, doch keine für die Replikation des Virus essentiellen Sequenzen enthält.

9. Element nach Anspruch 8, wobei das Virus ein humanes Immunschwäche-Virus ist.

10. Element nach Anspruch 9, wobei der Promotor der Immediate-Early-Promotor des humanen Cytomegalovirus ist.

11. Element nach einem der Ansprüche 8 bis 10, wobei die Protein-codierende Region die antigenen Haupt-Determinanten des Virus codiert.

12. Element nach einem der Ansprüche 8 bis 11, wobei die Protein-codierende Region ausreichend DNA enthält, um für alle der Determinanten zu codieren, die durch das virale Genom codiert sind.

13. Element nach einem der Ansprüche 8 bis 12, wobei die für die Replikation essentiellen Sequenzen die Long Terminal Repeat-Elemente und die Primer-Bindungsstellen sind.

14. Element nach einem der Ansprüche 8 bis 13, worin die Trägerpartikel Goldpartikel sind.

## Revendications

1. Utilisation de particules supports revêtues avec une construction de vaccin génétique pour fabriquer un médicament destiné à induire une réponse immunologique à un virus d'immunodéficience chez un primate, dans laquelle :
- la construction de vaccin génétique comprend un promoteur opérant dans des cellules du primate et une région codante de protéine codant pour des déterminants antigéniques dudit virus mais qui ne comprend pas de séquences essentielles pour la réplication du virus ;
- les particules supports sont de petite taille par rapport à la taille des cellules du primate ; et
- ladite réponse immunologique est induite en accélérant les particules supports revêtues dans la peau du primate.

2. Utilisation selon la revendication 1, dans laquelle le virus est le virus de l'immunodéficience humaine.

3. Utilisation selon la revendication 2, dans laquelle le promoteur est le promoteur précoce immédiat du cytomégalovirus humain.

4. Utilisation selon l'une quelconque des revendications précédentes, dans laquelle la région codante de protéine code pour les principaux déterminants antigéniques du virus.

5. Utilisation selon l'une quelconque des revendications précédentes, dans laquelle la région codante de protéine comprend de l'ADN en suffisance pour coder pour la totalité des déterminants codés par le génome viral.

6. Utilisation selon l'une quelconque des revendications précédentes, dans laquelle les séquences essentielles pour la réplication sont les éléments répétitifs terminaux longs et le site de liaison des amorces.

7. Utilisation selon l'une quelconque des revendications précédentes, dans laquelle les particules supports sont des particules d'or.

8. Dispositif de transfert génique à particules accélérées destiné à induire une réponse immunologique contre un virus d'immunodéficience chez un primate, ledit dispositif étant chargé avec des particules supports qui sont de petite taille par rapport à la taille des cellules du primate et qui sont revêtues avec une construction de vaccin génétique comprenant un promoteur opérant dans lesdites cellules et une région codante de protéine codant pour des déterminants antigéniques dudit virus mais ne comprenant pas de séquences essentielles pour la réplication du virus.

9. Dispositif selon la revendication 8, dans laquelle le virus est le virus de l'immunodéficience humaine.

10. Dispositif selon la revendication 9, dans laquelle le promoteur est le promoteur précoce immédiat du cytomégalovirus humain.

11. Dispositif selon l'une quelconque des revendications 8 à 10, dans laquelle la région codante de protéine code pour les principaux déterminants antigéniques du virus.

12. Dispositif selon l'une quelconque des revendications 8 à 11, dans laquelle la région codante de protéine comprend de l'ADN en suffisance pour coder pour la totalité des déterminants codés par le génome viral.

13. Dispositif selon l'une quelconque des revendications 8 à 12, dans laquelle les séquences essentielles pour la réplication sont les éléments répétitifs terminaux longs et le site de liaison des amorces.

14. Dispositif selon l'une quelconque des revendications 8 à 13, dans laquelle les particules supports sont des particules d'or.
